Europäisches Patentamt

(19) European Patent Office

Office européen des brevets

(11) Publication number: **0 017 651**
A1

# EUROPEAN PATENT APPLICATION

(22) Date of filing: 02.04.79

(21) Application number: **79100986.3**

(22) Date of filing: 02.04.79

(51) Int. Cl.³: **C 07 C 85/06,** C 07 C 85/08,
**B 01 J 23/74,** B 01 J 23/80

(43) Date of publication of application: **29.10.80**
**Bulletin 80/22**

(84) Designated Contracting States: **BE DE FR GB IT NL SE**

(71) Applicant: **THE DOW CHEMICAL COMPANY,**
**2030 Abbott Road Post Office Box 1967, Midland,**
**Michigan 48640 (US)**

(72) Inventor: **Habermann, Clarence Edward, 3615 Blarney,**
**Midland Midland Michigan (US)**

(74) Representative: **Casalonga, Alain et al, Bureau D.A.**
**Casalonga Lilienstrasse 77, D-8000 München 80 (DE)**

(54) **Improved method of producing amines from alcohols, aldehydes, ketones and mixtures thereof and catalyst used.**

(57) A method of producing amines, the method comprising contacting at reactive conditions at least one alcohol, aldehyde or ketone, or a mixture thereof, with an aminating agent in the presence of a catalyst, is improved by employing as the catalyst a compositon comprising cobalt, or nickel, copper and a third component selected from the group consisting of iron, zinc, zirconium and mixtures thereof. For example, polypropylene glycol is quantitatively aminated by contacting it with ammonia at about 175° C and in the presence of a catalyst comprising (metal basis) 30 mole percent cobalt, 63 mole percent copper and about 7 mole percent iron.

EP 0 017 651 A1

-1-

## IMPROVED METHOD OF PRODUCING AMINES
## FROM ALCOHOLS, ALDEHYDES, KETONES
## AND MIXTURES THEREOF AND CATALYST USED

This invention relates to an improved method of producing amines from alcohols, aldehydes, ketones and mixtures thereof. In one aspect, the invention relates to an ammonolytic method while in another aspect it relates to a catalyst useful therein.

The prior art describes numerous catalysts useful for the ammonolysis of an alcohol, and particularly primary and secondary alcohols, to the corresponding amine. Shirley and Speranza, U.S. Patent 3,128,311, teach a catalyst comprising about 50 to 90 weight percent nickel, about 10 to 50 weight percent copper and about 0.5 to 5 weight percent of an oxide selected from the class consisting of chromium oxide, titanium oxide, thorium oxide, magnesium oxide, zinc oxide and manganese oxide. Moss, U.S. Patent 3,152,998, teaches a catalyst characterized by having the composition calculated in mole percent on an oxide-free basis of 60 to 85 percent nickel, 14 to 37 percent copper and 1 to 5 percent chromium. While these and other catalysts have demonstrated

18,439A-F

ammonolytic utility, catalysts demonstrating superior ammonolytic activity are desirable for reasons of economy, efficiency and convenience.

The present invention is a method for preparing amines by contacting an alcohol, aldehyde or ketone with an aminating agent in the presence of a catalyst, where the catalyst is composed of nickel or cobalt, copper and iron, zinc or zirconium, characterized in that the catalyst comprises, calculated in mole percent and on an oxide-free basis:

(a) 20 to 90 percent cobalt and 8 to 72 percent copper, or 20 to 49 percent nickel and 36 to 79 percent copper, and

(b) 1 to 16 percent iron, zinc or zirconium.

The present invention also includes a catalyst for preparing amines from at least one alcohol, aldehyde or ketone, or a mixture thereof, the catalyst consisting of, calculated in mole percent and on an oxide-free basis:

(a) 20 to 90 percent cobalt;
(b) 8 to 72 percent copper; and
(c) 1 to 16 percent of iron, zinc or zirconium.

The present invention also includes a catalyst for preparing amines from at least one alcohol, aldehyde or ketone, or a mixture thereof,

18,439A-F

the catalyst consisting of, calculated in mole percent and on an oxide-free basis:

(a) 20 to 49 percent nickel;
(b) 36 to 79 percent copper; and
(c) 1 to 16 percent of iron, zinc or zirconium.

These compositions demonstrate superior ammonolytic activity thus allowing quantitative conversion of the alcohol to the corresponding amine at less rigorous method conditions. Moreover, these compositions demonstrate excellent selectivity and life.

The ammonolytic mechanism of this invention is believed to comprise:

(a) dehydrogenating the alcohol to the corresponding aldehyde or ketone;
(b) adding the aminating agent to the aldehyde or ketone to form an imine; and
(c) hydrogenating the imine to the corresponding amine.

Consequently, the catalytic compositions of this invention are also useful for the ammonolysis of aldehydes and ketones to the corresponding amines.

The preferred compositions of the present invention are those comprising, in mole percent and on an oxide-free basis:

18,439A-F

(a)   30 to 80 percent cobalt; 15 to 60 percent copper, and 2 to 14 percent iron, zinc or zirconium, and

(b)   30 to 49 percent nickel; 36 to 69 percent copper, and 1 to 16 percent iron, zinc or zirconium.

The especially preferred compositions are those comprising (same basis):

40 to 70 percent cobalt;
20 to 50 percent copper, and
4 to 12 percent iron, zinc or zirconium.

Compositions wherein the third component comprises only zirconium are particularly preferred.

These compositions can be used either unsupported or supported.  Typical supports include: alumina, silica, zirconia, zircon (a mixture of zirconia and silica), magnesia, and various cation exchange resins, especially those containing a sulfonated styrene-divinylbenzene copolymer matrix. If the composition is supported, the metal loading (on an oxide-free basis) is usually at least about 0.5 percent and preferably at least about 10 percent of the total weight (support plus composition).  The maximum metal content can vary to convenience but it is generally about 50 weight percent and preferably about 20 weight percent.

The catalytic compositions of this invention are readily prepared by any number of different methods but are typically prepared by first precipitating

18,439A-F

the metal components from their salts, e.g., nitrates, chlorides, sulfates, acetates, etc., in a basic, aqueous solution, e.g., sodium or ammonium carbonate, sodium or potassium hydroxide, alkali or alkaline earth metal oxalates, silicates or aluminates, etc. The metal precipitate is then washed, filtered and dried at an elevated temperature, e.g., 60°-180°C, and the dry precipitate is then decomposed at a temperature between about 200°C and about 400°C for a suitable period of time, e.g., 2 hours, to the corresponding oxides. If desired, preparation of the composition can commence with commercially available oxides rather than first preparing the oxides as here described. The resulting oxide mixture is then reduced with hydrogen, sodium borohydride, hydrazine, a reducing metal of greater oxidation potential than cobalt, such as carbon monoxide or some other suitable reducing agent. The degree of reduction is temperature dependent but generally, the first two components (cobalt and copper, or nickel and copper) are reduced to the active metal while the third component, i.e., zinc, iron, zirconium or a mixture thereof, remains an oxide. When this reduction is with hydrogen, a temperature between about 150°C and about 250°C for about 6 to 7 hours is usually adequate. The reduced catalyst is thereafter generally handled in the absense of air. If a supported catalyst is desired, the metal salts can be precipitated directly upon or with the carrier (support).

These compositions can also be prepared from suitable alloys of the three components and at least one leachable fourth component. For example,

18,439A-F

an alloy of either cobalt or nickel, copper, zirconium and aluminum can be formed and subjected to caustic whereby the aluminum is leached from the alloy. The resulting Raney-like structure is then ready for use.

A catalytic amount of the composition is required for the practice of this invention. The minimum amount of catalyst required will vary with the method reagents and conditions, but a typical minimum amount is about 0.1 weight percent, and preferably about 1 weight percent, based on the weight of the starting materials. Practical considerations, such as convenience, catalyst recovery and economy, are the only limitations upon the maximum amount of catalyst that can be used, although these considerations prefer a maximum of about 25 weight percent, and most preferably of about 10 weight percent.

Any alcohol that can be used in known ammonolytic methods can be used in the practice of this invention. These alcohols comprise a wide variety of hydroxy-containing materials. Representative alcohols include: primary and secondary alcohols, such as alkanols of 1 to about 18 carbon atoms, e.g., methanol, n-propanol, isopropanol, n-butanol, sec-butanol, isobutanol, the isomeric pentanols and hexanols, 2-ethylhexanol, tridecanol, stearyl alcohol, etc.; cycloalkanols of 5 to 12 carbon atoms, e.g., cyclohexanol, cycloheptanol, etc.; aralkanols of 7 to about 40 carbon atoms, e.g., benzyl alcohol, 2-phenyl ethanol, etc.; polyhydric alcohols of 2 to about 15 carbon atoms, e.g., ethylene glycol, propylene glycol, 1,3-butanediol, 1,4-butanediol, 1,5-pentanediol, hexamethylene glycol, decamethylene glycol, 1,12-dihydroxyoctadecane, glycerol, etc.; polymeric polyhydric alcohols, e.g., polyvinyl alcohol; glycol ethers and polyalkylene glycol ethers, e.g., methyl glycol, ethyl glycol, butyl glycol, diethylene glycol, triethylene glycol, tetraethylene glycol, higher polyethylene glycols, dipropylene glycol, tripropylene glycol, polypropylene glycol ether, polybutylene glycol ether, etc.; aminated alcohols, such as alkanolamines, e.g., ethanolamine, n-propanolamine, isopropanolamine, hexanolamine, diethanolamine, diisopropanolamine, dimethylethanolamine, etc.; and aminated polyhydric alcohols and glycol ethers, e.g., aminated polyethylene glycol, etc. Other suitable hydroxy-containing compounds are disclosed in U.S. Patents 3,347,926; 3,654,370 and 4,014,933.

Any aldehyde or ketone that can be produced from the dehydrogenation of the alcohols here used can also be used in the practice of this invention. Representative aldehydes include: methanal, ethanal, propanal, butanal, cyclohexanal, benzylaldehyde, and aldehydes prepared from the dehydrogenation of polyhydric alcohols, polymeric polyhydric alcohols, glycol ethers and polyalkylene glycol ethers, aminated alcohols, aminated polyhydric alcohols and glycol ethers. Representative ketones include: propanone, butanone, 2-pentanone, 3-pentanone, 3-methyl-2-butanone, 1-phenyl-2-propanone, acetophenone, n-butyrophenone, benzophenone, 3-nitro-4'-methyl-benzophenone, and ketones prepared from the dehydrogenation of polyhydric alcohols, polymeric polyhydric alcohols, glycol ethers and polyalkylene glycol ethers, aminated alcohols, aminated polyhydric alcohols and glycol ethers.

18,439A-F

As used herein, "at least one alcohol, aldehyde or ketone, or a mixture thereof", means that these compounds can be used either singly (which is preferred), or as a mixture of one or more alcohols, aldehydes or ketones, or a mixture of any two or all three of these types of compounds.

"Alcohol" includes those compounds containing both a hydroxy function and a carbonyl function and "aldehyde" includes those compounds without a hydroxy function but containing both an aldehyde carbonyl and a ketone carbonyl. Alcohols are preferred to aldehydes and aldehydes are preferred to ketones.

The aminating agents of this invention are ammonia or primary or secondary amines. The primary and secondary amines generally have alkyl radicals of 1 to about 12 carbon atoms or cycloalkyl radicals of 5 to 8 carbon atoms or aralkyl radicals of 7 to about 40 carbon atoms and include such compounds as: methylamine, dimethylamine, ethylamine, diethylamine, n-butylamine, sec-butylamine, isobutylamine, ethylenediamine, benzylamine, etc. Other suitable amines include cyclic amines which can contain hetero atoms other than nitrogen, such as oxygen, and these compounds include: morpholine, pyrrolidine, piperidine, piperizine, etc. When ammonia is the aminating agent, primary amines are obtained; when a primary amine is the aminating agent, secondary amines are obtained; when a secondary amine is the aminating agent, tertiary amines are obtained. These aminating agents, like the alcohols, aldehydes and ketones, can also be used either singly (which is preferred) or in

18,439A-F

combination with one another. Primary amines are preferred to secondary amines and ammonia is preferred to primary amines.

Stoichiometric amounts of alcohol and aminating agent are required for the practice of this invention. However, for reasons of convenience and efficiency, it is preferable to practice this invention with a stoichiometric excess of aminating agent to alcohol. Typically, a minimum aminating agent:alcohol mole ratio of about 1:1 and preferably of about 5:1 is employed. Practical considerations, such as economy and reaction equipment used, are the only limitations upon the maximum ratio, but these considerations prefer a mole ratio of about 200:1 and more preferably of about 100:1. The typical aminating agent:aldehyde, ketone or mixture mole ratios are generally the same as the here recited aminating agent:alcohol mole ratios.

The method of this invention generally employs hydrogen. The amount of hydrogen used can vary to convenience, but a typical minimum hydrogen: alcohol mole ratio is at least about 0.1:1 and preferably at least about 1:1. A typical maximum mole ratio is about 50:1 and preferably about 20:1. Again, the typical hydrogen:aldehyde, ketone or mixture mole ratios are generally the same as the here recited hydrogen:alcohol mole ratios.

Although conventional method conditions can here be used, the superior catalytic activity of this invention's composition permits the ammonolytic process to proceed at lower temperatures and pressures.

18,439A-F

For example, this invention can be practiced at a temperature of at least about 75°C although preferred reaction rates are obtained at a temperature of at least about 120°C. Pressures are of course dependent upon temperature but a minimum pressure of about 1000 psi (70.5 kg/cm$^2$) can be used although a minimum pressure of about 1500 psi (106 kg/cm$^2$) is preferred. Again, practical considerations are the only limitations upon the maximum temperature and pressure, but a maximum temperature of about 400°C and a maximum pressure of about 10,000 psi (705 kg/cm$^2$) are preferred. A more preferred maximum temperature is about 250°C and a more preferred maximum pressure is about 6000 psi (423 kg/cm$^2$).

The invention can be practiced on either a continuous or batch operation, in both the liquid and gas phases, and either neat or in the presence of an inert solvent. By "inert" is meant that the solvent is essentially nonreactive with the method reagents and products under the conditions used. Exemplary solvents include tertiary alcohols, such as tertiary butanol and tertiary amylalcohol; ethers, such as diethylether and dimethylether; aliphatic and aromatic hydrocarbons, such as hexane, heptane, cyclohexane, benzene, toluene and xylene; halogenated aliphatic and aromatic hydrocarbons, such as chloroethane, dichloroethane, chlorobenzene, o-dichlorobenzene and chloroform; and nonreactive tertiary amines, such as pyridine, picoline and lutadine. Moreover, this method can be practiced in either the presence or absence of water, although if water is present, it is preferred that it is not present in amounts greater than about 50 weight percent of the alcohol, aldehyde, ketone or mixture.

18,439A-F

Whether the amines produced by this invention are primary, secondary or tertiary depends not only upon the aminating agent employed, but also upon the particular method conditions used. Short contact time, e.g., between about 0.1 seconds and about 15 minutes, excess ammonia and low temperature and pressure generally favor the production of primary amines. As the aminating agent:alcohol, aldehyde, ketone or mixture mole ratio decreases and/or the temperature increases and/or the contact time increases, secondary and tertiary amines form a larger percentage of the final product. However, longer reaction time favors greater amination of the alcohol. Accordingly, by appropriate selection of aminating agent and process conditions, it is possible to change the proportions of primary, secondary and tertiary amines prepared.

The following examples illustrate the invention. Unless otherwise noted, all parts and percentages are by weight.

Catalyst Preparation

The catalysts used in the following examples and controls (unless indicated otherwise) were prepared by adding a solution (0.5 molar) of the corresponding nitrates and a solution (0.5 molar) of ammonium carbonate simultaneously to a reaction flask. The pH (about 6.5) was controlled by the flow rate of the respective solutions. The resulting precipitated carbonates were washed, filtered and subsequently dried at about 60°C. The dry carbonates where then decomposed at about 250°C for about one hour to the corresponding oxides. The oxides were subsequently reduced with hydrogen at a temperature of about 200°C

18,439A-F

for about 6 hours.   The reduced catalyst was then handled in the absence of air.


## Procedure

Reduced catalyst (15 g) prepared as above and polypropylene glycol (15 g) of about 400 molecular weight were charged under a protective atmosphere of nitrogen to a rocking autoclave.   The autoclave was then charged with ammonia (60 g) and hydrogen (500 psig) and the autoclave contents were subsequently heated for about 5 hours at about 180°C.   Afterwards, the autoclave was cooled, vented and the catalyst removed by filtration.   The catalyst was then washed with dry methanol and the washings combined with the remaining autoclave contents.   Methanol and water were then removed from these contents by a rotary evaporator and the resulting product analyzed either by vapor phase chromatography or titration with hydrochloric acid.


## Examples 1-32

The following examples were performed with catalysts prepared as above and by the above procedure. The figures preceding each catalyst component represent that component's mole percent of the catalyst on an oxide-free basis.   The figures reported at the percent amination column represent the weight percent of polypropylene glycol aminated.

0017651

| Example | Catalyst | | | Amination Wt. % |
|---|---|---|---|---|
| 1 | 20-Co, | 64-Cu, | 16-Zn | 26 |
| 2 | 30-Co, | 56-Cu, | 14-Zn | 36 |
| 3 | 40-Co, | 48-Cu, | 12-Zn | 29 |
| 4 | 50-Co, | 40-Cu, | 10-Zn | 27 |
| *5 | 60-Co, | 32-Cu, | 8-Zn | 28 |
| 6 | 70-Co, | 24-Cu, | 6-Zn | 27 |
| 7 | 80-Co, | 16-Cu, | 4-Zn | 23 |
| 8 | 90-Co, | 8-Cu, | 2-Zn | 13 |
| 9 | 20-Co, | 72-Cu, | 8-Zr | 46 |
| 10 | 30-Co, | 63-Cu, | 7-Zr | 46 |
| 11 | 40-Co, | 54-Cu, | 6-Zr | 52 |
| 12 | 50-Co, | 45-Cu, | 5-Zr | 53 |
| 13 | 60-Co, | 36-Cu, | 4-Zr | 47 |
| 14 | 70-Co, | 27-Cu, | 3-Zr | 45 |
| 15 | 80-Co, | 18-Cu, | 2-Zr | 45 |
| 16 | 90-Co, | 9-Cu, | 1-Zr | 27 |
| 17 | 20-Co, | 64-Cu, | 16-Fe | 12 |
| 18 | 30-Co, | 56-Cu, | 14-Fe | 20 |
| 19 | 40-Co, | 48-Cu, | 12-Fe | 26 |
| 20 | 50-Co, | 40-Cu, | 10-Fe | 33 |
| 21 | 60-Co, | 32-Cu, | 8-Fe | 35 |
| 22 | 70-Co, | 24-Cu, | 6-Fe | 28 |
| 23 | 80-Co, | 16-Cu, | 4-Fe | 19 |
| 24 | 90-Co, | 8-Cu, | 2-Fe | 31 |
| 25 | 20-Co, | 72-Cu, | 8-Fe | 54 |
| 26 | 30-Co, | 63-Cu, | 7-Fe | 60 |
| 27 | 40-Co, | 54-Cu, | 6-Fe | 52 |
| 28 | 50-Co, | 45-Cu, | 5-Fe | 55 |
| 29 | 60-Co, | 36-Cu, | 4-Fe | 51 |
| 30 | 70-Co, | 27-Cu, | 3-Fe | 35 |
| 31 | 80-Co, | 18-Cu, | 2-Fe | 51 |
| 32 | 90-Co, | 9-Cu, | 1-Fe | 51 |
| 33 | 30-Ni, | 56-Cu, | 14-Zn | 43.1 |
| 34 | ~50-Ni, | 45-Cu, | 5-Zr | 77.6 |
| 35 | 30-Ni, | 63-Cu, | 7-Fe | 76.9 |
| **Control** | | | | |
| **A | 80-Ni, | 16-Cu, | 4-Zn | 11.8 |
| B | 80-Ni, | 18-Cu, | 2-Zr | 29.5 |
| C | 90-Ni, | 9-Cu, | 1-Fe | 60.9 |

*Contact time of about 6.25 hours

**Temperature about 170°C

18,439A-F

The control catalysts here used correspond to those known in the art, i.e., catalysts wherein nickel is present in at least about 50 mole percent of the catalyst. A comparison of the example and control data demonstrates the superior activity of the former over the latter.

Example 36

A rocking, 300 cc autoclave was charged with monoethanolamine (20 g), ammonia (75 g), hydrogen at 500 psig (35.3 kg/cm²) and a catalytic composition (8 g) comprising cobalt (30 mole percent), copper (63 mole percent) and $Fe_2O_3$ (7 mole percent). The charged autoclave was heated to about 180°C and held thereat for 5 hours while continually rocked. Thereafter excess ammonia was vented, the contents filtered and subsequently analyzed by gas chromatography. 67 Percent of the starting monoethanolamine was converted and the product mix contained 43 weight percent ethylendiamine.

Example 37

The same procedure and proportions of components were used as for Example 36, except that the catalyst contained 30 mole percent nickel instead of 30 mole percent cobalt. Conversion of the monoethanolamine was 30 percent and the final product contained 56 weight percent ethylendiamine.

Example 38

A 0.75 inch (1.91 cm) stainless steel tube, one foot long (30.5 cm) was packed with a catalytic composition (66 g) comprising cobalt (50 mole percent), copper (45 mole percent) and $ZrO_2$ (5 mole percent) and

18,439A-F

was heated by a hot oil bath. The results of several runs at various temperatures are tabulated below. A mixture of monoethanolamine (MEA) and ammonia ($NH_3$:MEA mole ratio of about 7) with a blanket of hydrogen at 500 psig (35.3 kg/cm$^2$) was pumped through the packed tube at 1500 psig (106 kg/cm$^2$) and 180 cc/hr. Contact time was about 10 minutes.

Results of Continuous
Ammonolytic Operation
with Co-Cu-Zr Composition

| Run | Temp. (°C) | Conversion (wt. %) | EDA Yield[1] (wt. %) |
|-----|-----|-----|-----|
| A | 150 | 32 | 82 |
| B | 175 | 78 | 70 |
| C | 200 | 82 | 40 |

[1]Ethylenediamine yield based on the weight of the product mix.

Control D

Example 38 was repeated except that a commercially available catalytic composition (146 g) comprising cobalt (70-75 mole percent), copper (20-25 mole percent) and chromium oxide (1-5 mole percent) was substituted for the catalytic composition of Example 38. The results are tabulated below.

18,439A-F

0017651

Results of Continuous
Ammonolytic Operation
with Co-Cu-Cr Composition

| Run | Temp. (°C) | Conversion (wt. %) | EDA Yield[1] (wt. %) |
|-----|-----------|--------------------|----------------------|
| A   | 150       | 10                 | 76                   |
| B   | 175       | 56                 | 52                   |
| C   | 200       | 56                 | --                   |

[1]Ethylenediamine yield based on the weight of the product mix.

18,439A-F

CLAIMS:

1. A method for preparing amines by contacting an alcohol, aldehyde or ketone with an aminating agent in the presence of a catalyst, where the catalyst is composed of nickel or cobalt, copper and iron, zinc or zirconium, characterized in that the catalyst comprises, calculated in mole percent and on an oxide-free basis:

(a) 20 to 90 percent cobalt and 8 to 72 percent copper, or 20 to 49 percent nickel and 36 to 79 percent copper, and

(b) 1 to 16 percent iron, zinc or zirconium.

2. The method of Claim 1 wherein the catalyst comprises, calculated in mole percent and on an oxide-free basis, about:

(a) 30 to 80 percent cobalt;
(b) 15 to 60 percent copper; and
(c) 2 to about 14 percent of iron, zinc or zirconium.

3. The method of Claim 1 wherein the catalyst comprises, calculated in mole percent and on an oxide-free basis, about:

18,439A-F

- 2 -

0017651

(a)  40 to 70 percent cobalt;

(b)  20 to 50 percent copper; and

(c)  4 to 12 percent of iron, zinc or zirconium.

4.  The method of Claim 1 wherein the catalyst comprises, calculated in mole percent and on an oxide-free basis, about:

(a)  30 to 49 percent nickel;

(b)  36 to 69 percent copper; and

(c)  1 to 16 percent of iron, zinc or zirconium.

5.  The method of Claim 1 wherein the contacting is performed at a temperature of at least 75°C and a pressure of at least 1000 psi (70.5 kg/cm$^2$).

6.  The method of Claim 5 wherein the contacting is performed at a temperature of from 120°-250°C.

7.  The method of Claim 5 wherein the contacting is performed under a pressure of from 1000 to 6000 psi (70.5 to 423 kg/cm$^2$).

8.  A catalyst for preparing amines from at least one alcohol, aldehyde or ketone, or a mixture thereof, the catalyst consisting of, calculated in mole percent and on an oxide-free basis:

(a)  20 to 90 percent cobalt;

(b)  8 to 72 percent copper; and

(c)  1 to 16 percent of iron, zinc or zirconium.

18,439A-F

0017651

9. The catalyst of Claim 8 consisting of:

(a) 30 to 80 percent cobalt;
(b) 15 to 60 percent copper; and
(c) 2 to 14 percent of iron, zinc or
zirconium.

10. The catalyst of Claim 8 consisting of:

(a) 40 to 70 percent cobalt;
(b) 20 to 50 percent copper; and
(c) 4 to 12 percent of iron, zinc or
zirconium.

11. A catalyst for preparing amines from
at least one alcohol, aldehyde or ketone, or a mixture
thereof, the catalyst consisting of, calculated in
mole percent and on an oxide-free basis:

(a) 20 to 49 percent nickel;
(b) 36 to 79 percent copper; and
(c) 1 to 16 percent of iron, zinc or
zirconium.

18,439A-F

## DOCUMENTS CONSIDERED TO BE RELEVANT

| Category | Citation of document with indication, where appropriate, of relevant passages | Relevant to claim |
|---|---|---|
| | GB - A - 936 322 (JEFFERSON CHEMICAL COMP.)<br>* Page 1, lines 24-64 *<br><br>-- | 1-11 |
| | CHEMICAL ABSTRACT, vol. 89, no. 21, November 20, 1978, abstract 179532n.<br>Columbus, Ohio, USA<br><br>& HU - A - 14 908 (PETI NITRO-GENMUVEK) (27-05-1978)<br><br>-- | |
| A | GB - A - 1 195 287 (B.A.S.F. A.G.)<br>* Page 1, lines 48-64; examples *<br><br>-- | 1 |
| A | US - A - 4 113 662 (R.G. WALL)<br>* Column 4, line 48 - column 5, line 20 *<br><br>-- | 8-10 |
| A | CHEMICAL ABSTRACTS, vol.89, no. 24, December 1978,<br>page 152, abstract 200204u<br>Columbus, Ohio, USA<br>ZHOROV, Yu,M. et al. "Purification of straight-run gasoline from sulfur and nitrogen compounds".<br><br>& SU - A - 622 836 (GUBKIN, I.M., INSTITUTE OF THE PETROCHEMICAL AND GAS INDUSTRY, MOSCOW)<br><br>-- | 11 |
| A | US - A - 4 083 799 (J.H. ESTES)<br>* Column 3, lines 50-66; alloy 7 *<br>-- ./. | 11 |

**CLASSIFICATION OF THE APPLICATION (Int. Cl. 3)**

C 07 C 85/06
          85/08
B 01 J 23/74
          23/80

**TECHNICAL FIELDS SEARCHED (Int.Cl. 3)**

C 07 C 85/00
B 01 J 23/00

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 26-11-1979 | PAUWELS |

EPO Form 1503.1   06.78

European Patent
Office

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| A | US - E - 27 296 (YATARO ICHIKAWA) <br> * Example E; table 5: E-2 * <br><br> ---- | 8-10 | |
| | | | **TECHNICAL FIELDS SEARCHED (Int. Cl. 3)** |